# EUROPEAN PATENT APPLICATION

(11) **EP 4 678 207 A1**
(43) Date of publication of application: **14.01.2026**
(21) Application number: 24767294.2
(22) Date of filing: 01.02.2024
(51) Int. Cl.: A61M 5/32, A61B 50/30, A61L 2/10

(54) **USED NEEDLE SEPARATING DEVICE**

(30) Priority: 08.03.2023 KR 20230030531; 04.12.2023 KR 20230173119
(71) Applicant: Mobiu Co., Ltd., Seoul 08859 (KR)
(72) Inventor: KIM, Hyung-Seok, Seoul 08862 (KR)
(74) Representative: Rau, Schneck & Hübner Patentanwälte Rechtsanwälte PartGmbB
(86) International application number: PCT/KR2024/001504
(87) International publication number: WO 2024/186005

(57) **Abstract**

The present invention relates to a used needle separating device in which engagement teeth for drive rotation are formed along the circumference on one side, and a non-engagement portion for accommodating a used needle and engagement teeth for separating (pulling out) the used needle are formed along the circumference on the other side.

## Description

### Technical Field

The present invention relates to a used needle separating device in which engagement teeth for drive rotation are formed along a circumference on one side thereof, and a non-engagement portion for accommodating the used needle and engagement teeth for separating (pulling out) the used needle are formed along a circumference on an opposite side thereof.

### Background Art

Syringes include various types of syringes, such as general-purpose syringes, blood collection syringes, insulin syringes, intravenous catheters, and blood collection-dedicated vacuum needles, depending on the using status.

In addition, a needle hub of the syringe is formed as an integrated type, an insertion type, a Luer lock type or the like according to an end part of a syringe body.

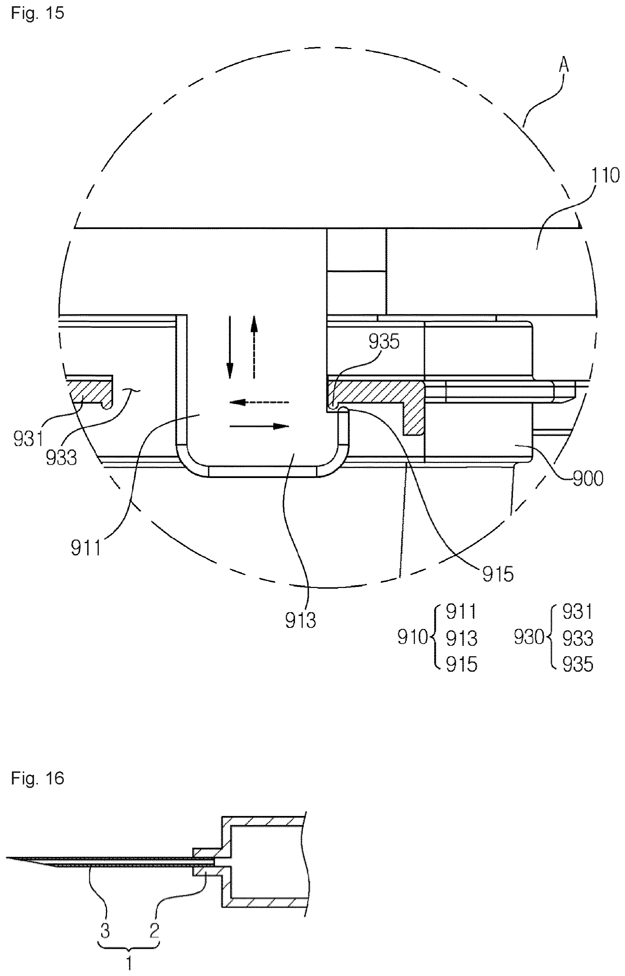

In addition, referring to FIG. 16, a needle hub 1 for a syringe is configured such that when a needle 3 is fixed to a hub 2, only one part of the needle 3 is inserted into the hub 2 and the inserted part may be coated with an adhesive.

In addition, the needle 3 of the needle hub 1 may also have various lengths and thicknesses.

In addition, the diameter of the hub 2 may have a different diameter (large diameter, small diameter and the like) and a different shape (cylindrical, cross-shaped) depending on the type of syringe.

A conventional a used syringe needle separating device is disclosed, for example, in the patent document (Korean Registered Patent Publication No. 10-1588170).

According to the used syringe needle separating device of the patent document, a syringe needle having passed through a pair of rotating needle separating gears is pulled back into a pair of needle processing gears to separate the syringe needle from a syringe.

However, the used syringe needle separating device of the patent document has problems as follows.

First, in the case that the needle has an appropriate length as in the patent document, because a needle tip is pulled from a point touching the engaged part of the needle processing gears, the operation is required as long as the length of the needle.

Second, in the case that the needle has a short length as in an insulin syringe, the needle may not be separated because the needle in inserted into a needle through-hole, but the needle tip fails to touch the engaged part of the needle processing gears. Third, in the case that the needle has a long length, because a user is required to hold a syringe body to proceed even when the needle tip touches the engaged part of the needle processing gears, it may be very inconvenient and it may take longer time to separate the needle.

Fourth, depending on the diameter or shape of the needle hub, the syringe may not be mounted on a syringe mounting portion.

Since the conventional automatic syringe needle separation and collection device may not handle needles and needle hubs to be suitable for types thereof, equipment for individually handling the needles and the needle hubs is required.

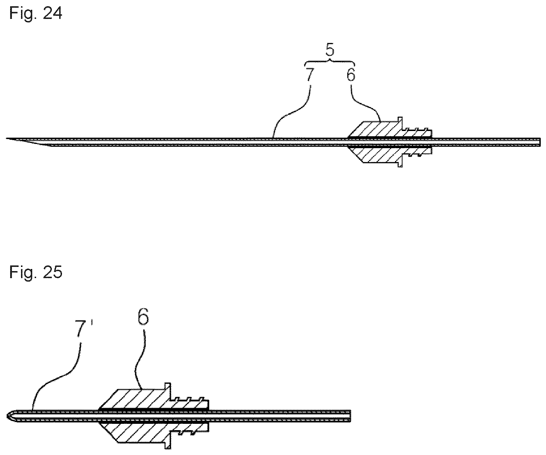

Meanwhile, according to a blood collection-dedicated needle or a catheter-dedicated needle, for example, as the blood collection-dedicated needle hub 5 shown in FIG. 24, a needle 7 passes through a hub 6, and the space between the hub 6 and the needle 7 is fixed using an adhesive.

Because the blood collection-dedicated needle hub 5 or the catheter-dedicated needle hub is fixed in a penetrating state unlike the general syringe having a needle tip portion partially inserted (see FIG. 16), the needle cannot be pulled out due to strong adhesion of the adhesive, and accordingly, a needle 7' partially cut without being completely separated remains as shown in FIG. 25.

In addition, because the needle 7 passes through the hub 6, the needle 7 blocks the hub, and accordingly, only the hub 6 may not be cut using a knife or the like.

Accordingly, the blood collection-dedicated needle hub 5 or the needle hub for IV catheter is required to be discarded while having the needle 7' without being completely removed.

### (Document of Related Art)

(Patent Document 1) Korean Registered Patent Publication No. 10-1588170 Disclosure

### Technical Problem

An object of the present invention is to solve the above-mentioned problems and other problems.

Another object of the present invention is to provide a used syringe needle separating device capable of quickly separating a needle regardless of a shape of a needle hub or a length of the needle.

Another object of the present invention is to provide a used syringe needle separating device capable of easily pulling out a needle dedicated for blood collection or catheter from a hub.

### Technical Solution

One aspect of the present invention to achieve the above or other objects provides a used needle separating device including: a case formed therein with an insertion port for a used syringe needle; first and second gears supported by a gear support bracket mounted on the case to separate the used syringe needle inserted into the insertion port; and a drive portion for rotating the first and second gears, wherein In the first and second gears, first and second engagement teeth for drive rotation are formed along one circumference thereof, and first and second non-engagement portions for accommodating the used needle and first and second engagement teeth for engaging and separating the used needle are formed along an opposite circumference thereof.

### Advantageous Effects

The advantageous effects of the used needle separating device according to the present invention will be described as follows.

According to at least one of the embodiments of the present invention, the used needle separating device may be provided in which the first and second non-engagement portions for accommodation are formed on the first and second gears for separating the needle, and accordingly, the needle tip is placed directly on the first and second engagement teeth for separation, so that the needle separation time can be significantly reduced regardless of length of the needle.

According to at least one of the embodiments of the present invention, the used needle separating device may be provided in which the guide jig portion according to the shape of the needle hub are provided to dispose the needle hub directly on the first and second engagement teeth for separation, so that the needle can be quickly separated regardless of shape of the needle hub.

According to at least one of the embodiments of the present invention, the used needle separating device may be provided in which the non-engagement portions for accommodation are formed in both directions at 180 degrees, so as to be rotated only within 180 degrees, so that power consumption can be reduced.

According to at least one of the embodiments of the present invention, the used needle separating device may be provided in which a start button is disposed on one side of the case for the insertion port to allow the user to insert the needle hub into the insertion port, push the syringe body laterally and then press the start button to operate, so that separation efficiency can be increased.

According to at least one of the embodiments of the present invention, the used needle separating device may be provided in which a dedicated needle hub is inserted inside a high frequency coil for heating metal and heated to transmit heat from the heated needle to the adhesive, thereby reducing adhesion strength, so that the entire needle can be easily pulled out from the dedicated needle hub.

According to at least one of the embodiments of the present invention, the used needle separating device may be provided in which the first start switch is simultaneously touched while inserting the needle hub to sequentially apply power to the high frequency induction heater and the driving portion, so that convenience in use can be further improved.

Further scope of applicability of the present invention will be apparent from the detailed description below. However, since various changes and modifications within the spirit and scope of the present invention will be apparent to those skilled in the art, it will be understood that the detailed description and specific embodiments, such as preferred embodiments of the present invention, are merely given as an example.

### Description of Drawings

FIG. 1 is an exterior perspective view showing a set of a used needle separating device 10 according to the preferred embodiment of the present invention.
FIG. 2 is a perspective view shown by removing a cover case 130 of FIG. 1.
FIG. 3 is an exploded perspective view showing main parts of FIG. 2.
FIG. 4 is a plan view of a bottom case 110.
FIG. 5 is an exterior perspective view of the cover case 130.
FIG. 6 is an enlarged perspective view showing first and second gears 200a and 200b.
FIGS. 7 and 8 are front and rear perspective views showing a holder portion 400.
FIG. 9 is a front perspective view showing a guide jig portion 500.
FIG. 10 is a rear view showing a state in which the holder portion 400 and the guide jig portion 500 are combined.
FIG. 11 shows front views of operating positions of the guide jig portion 500 with respect to the holder portion 400.
FIG. 12 shows views of a process for separating a needle having a general length.
FIG. 13 shows views of a process for separating a needle having a very short length.
FIG. 14(a) is a view showing a used syringe inserted into an insertion port 131 and touching a start button 330, and FIG. 14(b) is a view showing the used syringe dropped by its own weight after a used syringe needle is completed in separation.
FIG. 15 is a sectional view showing an attachment/detachment operation between a needle collection container 900 and the bottom case 110.
FIG. 16 is a sectional view showing a needle hub 1 for a general syringe.
FIG. 17 is an exterior perspective showing a used needle separating device 1000 according to another preferred embodiment of the present invention.
FIG. 18 is a perspective view shown by removing a cover case 130 of FIG. 17.
FIG. 19 is an exploded perspective view showing main parts of FIG. 18.
FIG. 20 is a sectional view showing the main parts of FIG. 18.
FIG. 21 is a left side view of FIG. 18.
FIGS. 22 and 23 are front and rear perspective views showing a holder portion 1400 of FIG. 19.
FIG. 24 is a sectional view showing a blood collection-dedicated needle hub 5.
FIG. 25 is a sectional view showing a state in which the needle of FIG. 24 is partially cut.

### Best Mode

### Mode for Invention

Hereinafter, the embodiments disclosed in the present specification will be described in detail with reference to the attached drawings. Regardless of the drawing numerals, identical or similar components are given the same reference numerals and redundant descriptions thereof will be omitted. The suffix such as "module" and "unit" for components used in the following description is given or mixed merely to facilitate description of the specification, and the suffix itself is not intended to give any meaning or function to differentiate the components with each other. Further, in the following description of the embodiments of the present invention, a detailed description of known functions and configurations incorporated herein will be omitted when it possibly makes the subject matter of the present disclosure unclear. In addition, the attached drawings are only intended to facilitate understanding of the embodiments disclosed in the present specification, and the technical idea disclosed in the present specification is not limited by the attached drawings. It will be understood to include all modifications, equivalents, or substitutes included in the idea and technical scope of the present invention.

The terms including an ordinal number such as first and second may be used to describe various components, however, these components are not limited by the above-mentioned terms. The terms are used only for the purpose of distinguishing one component from another component.

When one component is recited to be "connected" or "linked" to other component, it will be understood that the one component may be directly connected to or linked to the other component, but another component may be present therebetween. On the other hand, when it is mentioned that one component is "directly connected" or "directly linked" to other component, it will be understood that another component is not present therebetween.

The singular expression includes a plural expression unless the context clearly means otherwise.

Herein, it will be understood that the term such as "include" or "have" is intended to designate the presence of a feature, number, step, component, part or a combination thereof recited in the specification, and does not preclude the possibility of the presence or addition of one or more other features, numbers, steps, components, parts or combinations thereof.

In the drawings, sizes of components may be exaggerated or reduced for convenience of description. For example, the size and thickness of each component shown in the drawing are arbitrarily shown for convenience of description, and therefore the present invention is not necessarily limited to the shown drawing.

In some embodiments, where implementations are otherwise feasible, a specific process sequence may be performed in a sequence different from a described sequence. For example, two processes described sequentially may be performed substantially simultaneously, or may proceed in a reverse sequence to the described sequence.

In the following embodiments, when films, regions, components or the like are connected, it includes not only the case in which the films, regions or components are directly connected, but also the case in which other films, regions, or components are interposed and indirectly connected between the films, regions, and components. For example, when the present specification is configured that a film, region, component or the like is electrically connected, it includes not only the case in which the film, region, component or the like is directly electrically connected, but also the case in which another film, region, component or the like is interposed and indirectly electrically connected therebetween.

Hereinafter, an x-axis is defined as the forward-backward direction, a y-axis as the left-right direction, and a z-axis as the up-down direction.

### Embodiment

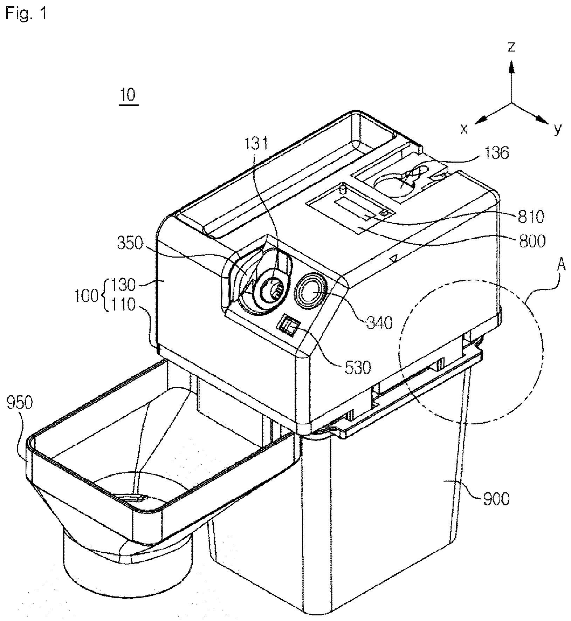

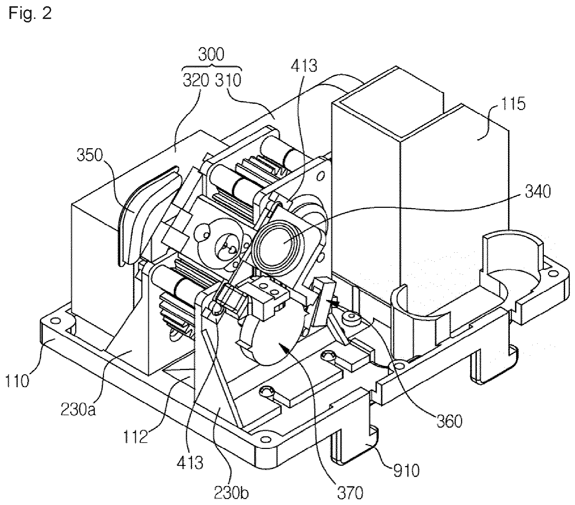

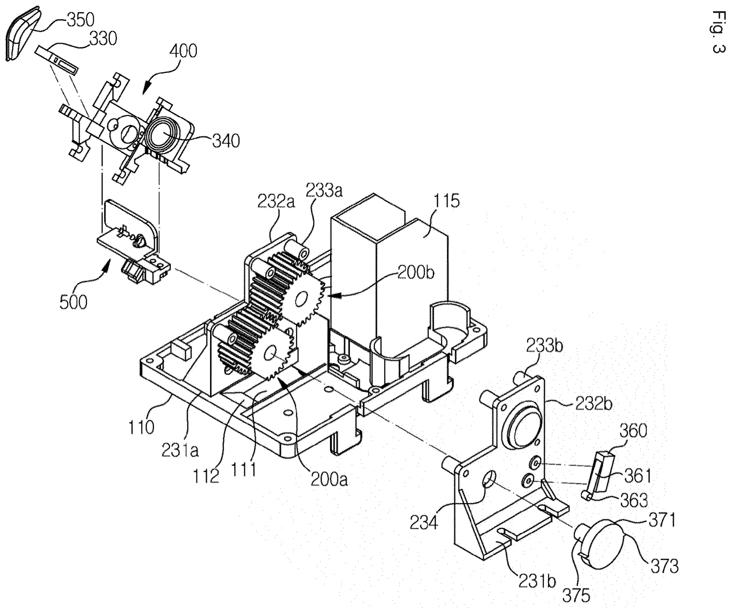

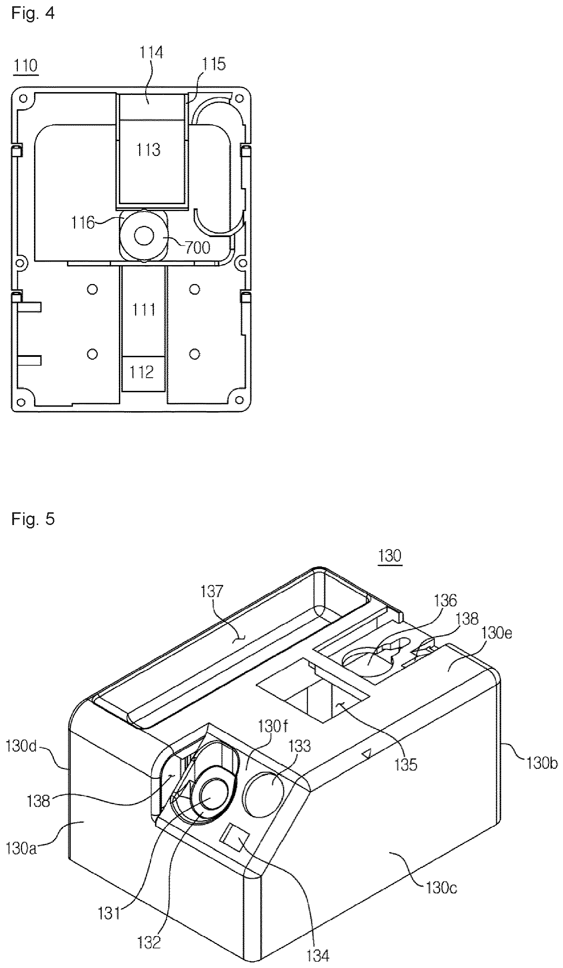

FIG. 1 is an exterior perspective view showing a set of a used needle separating device 10 according to the preferred embodiment of the present invention; FIG. 2 is a perspective view shown by removing a cover case 130 of FIG. 1; FIG. 3 is an exploded perspective view showing main parts of FIG. 2; FIG. 4 is a plan view of a bottom case 110; and FIG. 5 is an exterior perspective view of the cover case 130.

Referring to FIG. 1, the used needle separating device 10 according to the embodiment of the present invention may include a case 100. The case 100 may include a plate-shaped bottom case 110 and a cylinder-shaped cover case 130.

As shown in FIG. 2, the bottom case 110 may fix or support various parts. As shown FIGS. 3 and 4, the bottom case 110 may include a discharge hole 111 formed in a front center thereof. The discharge hole 111 may allow a separated needle to fall into a needle collection container 900. An inclined surface 112 inclined downward toward the discharge hole 111 may be formed in front of the discharge hole 111. The inclined surface 112 may guide the separated needle to slide down toward the discharge hole 111 when the separated needle falls and bounces.

As shown FIG. 4, the bottom case 110 may include an additional discharge hole 113 formed in a rear center thereof. The additional discharge hole 113 may pass a needle separated by manual work. Similar to the inclined surface 112, an additional inclined surface 114 inclined downward toward the additional discharge hole 113 may be formed in back of the additional discharge hole 113. As shown FIGS. 2 and 3, a shield wall 115 for covering left, right and front sides of the additional discharge hole 113 and the additional inclined surface 114 may protrude upward. The shield wall 115 may be a column having a rectangular C shape. The shield wall 115 may allow the manually separated needle, even upon bouncing, to fall into the additional discharge hole 113.

As shown FIG. 4, the bottom case 110 may include a light irradiation through-hole 116. The light irradiation through-hole 116 may be arranged between the discharge hole 111 and the additional discharge hole 113. The light irradiation through-hole 116 may serve as a passage through which light from a sterilizing lamp 700 is irradiated to the needle collection container 900. When power is applied to the sterilizing lamp 700, a UVC-LED may be turned on to sterilize and disinfect separated needles accumulated in the needle collection container 900.

As shown in FIG. 5, the cover case 130 may have a hexahedral shape for covering an upper part of the bottom case 110. The cover case 130 may include front, back, left, right and top plates 130a to 130e.

A corner between the front plate 130a and the top plate 130e may be formed as an inclined plate 130f pressed and recessed at a 45 degree angle.

An insertion port 131 may be formed in the inclined plate 130f. A needle hub of the used needle may be inserted and pass through the insertion port 131. A step 132 on which a body of a used syringe is caught may be formed around the insertion port 131.

A through-hole 133 in which a second start switch 340 is disposed may be formed in the inclined plate 130f.

A long hole 134 in which a grip portion 530 described later is slidably disposed may be formed in the inclined plate 130f.

A through-hole 138 may be formed on a side of the case in which the front plate 130a, the inclined plate 130f and the top plate 130f meet each other. The through-hole 138 may be adjacent to the insertion port 131 in a perpendicular direction. The through-hole 138 may be disposed therein with a start button 350 for touching a first start switch 330.

A through hole 135 in which a display window 810 of a counter 800 is disposed may be formed in the top plate 130e. The counter 800 may have a rectangular container installed above the sterilizing lamp 700. The rectangular container of the counter 800 may function to allow the separated needle to bounce backward and fall into the discharge hole 111. A metal plate (not shown) may be placed on a part in which the separated needle collides so as to prevent scratches and the like. In addition, when the needle is very long, such as 5 cm or longer, a tip of the needle touches the metal plate (not shown) and the separated tip of the needle is directed toward the floor, so as to function to collect the needle into the needle collection container 900. In addition, the metal plate (not shown) is preferably formed of stainless steel which prevents rust due to blood or drugs on the tip of the needle. A pulse-type counter 800 may be supplied with power and display a number, which increases by one count whenever the needle is separated, on the display window 810.

The top plate 130e may be formed therein with a manual needle separation penetration portion 136 and a manual scalpel blade separation penetration portion 138. The manual needle separation penetration portion 136 and the manual scalpel blade separation penetration portion 138 may be formed directly above the shield wall 115. The manual needle separation penetration portion 136 may be configured to separate the needle 3 when the needle hub 1 is inserted downward from above, pulled laterally and then bent. The manual scalpel blade separation penetration portion 138 may be configured to separate a scalpel blade when a fixed portion of a scalpel having the scalpel blade fixed thereto is inserted downward and pulled out.

A groove 137 may be formed on the top plate 130e to temporarily place a used syringe having a needle which is not separated.

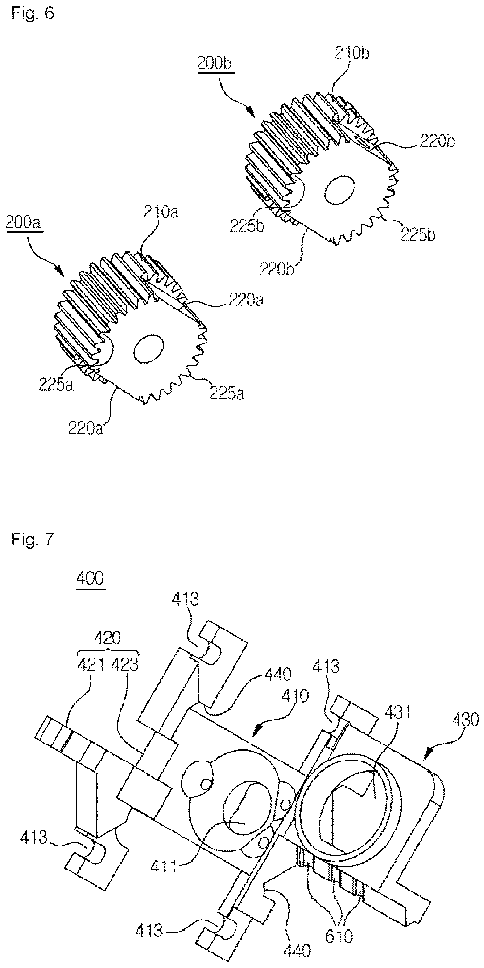

FIG. 6 is an enlarged perspective view showing first and second gears 200a and 200b of FIG. 3.

Referring to FIG. 6, the used needle separating device 10 according to the embodiment of the present invention may include the first and second gears 200a and 200b for separating (pulling out) a used needle. The first and second gears 200a and 200b may be a pair of spur gears.

The first and second gears 200a and200b serving as the core of the present invention may include first and second engagement teeth 210a and 210b for drive rotation formed along the entire circumferences thereof, respectively. The first and second engagement teeth 210a and 210b for drive rotation may engage and rotate the first and second gears 200a and200b. In the present embodiment, the first gear 200a may serve as a driving gear and the second gear 200b may serve as a driven gear, but may also serve reversely.

In addition, the first and second gears 200a and200b serving as the core of the present invention may include first and second non-engagement portions 220a and 220b for accommodation and first and second engagement teeth 225a and 225b for separation formed along opposite circumferences thereof, respectively. The first and second non-engagement portions 220a and 220b for accommodation may allow the used needle 3 to be accommodated to the first and second engagement teeth 225a and 225b for separation as close as possible. The first and second engagement teeth 225a and 225b for separation may engage and separate the accommodated used needle 3. The first and second non-engagement portions 220a and 220b for accommodation may be formed on only one side or may be formed at 180-degree intervals as in the present embodiment. In addition, an empty space between the first and second non-engagement portions 220a and 220b for accommodation may be arranged to face the insertion port 131 to communicate with each other.

The number of each of the first and second engagement teeth 210a and 210b for drive rotation of the present embodiment may be 30, each of the first and second non-engagement portions 220a and 220b for accommodation may be formed by removing six teeth from each of both sides, and the first and second engagement teeth 225a and 225b for separation each having nine teeth may separate the used needle 3.

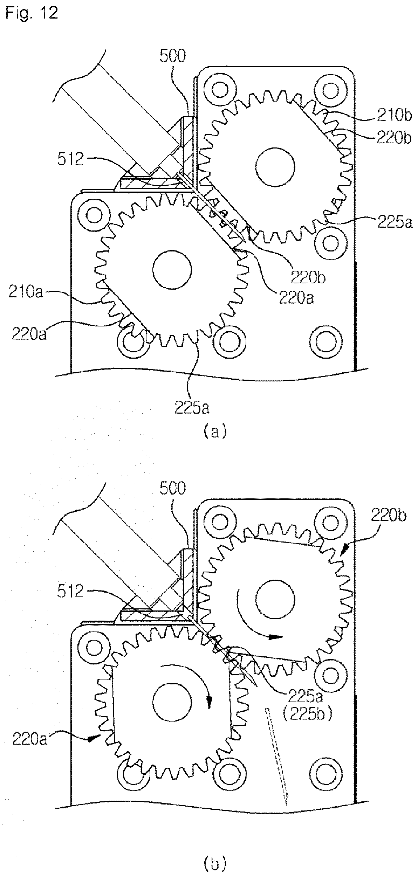

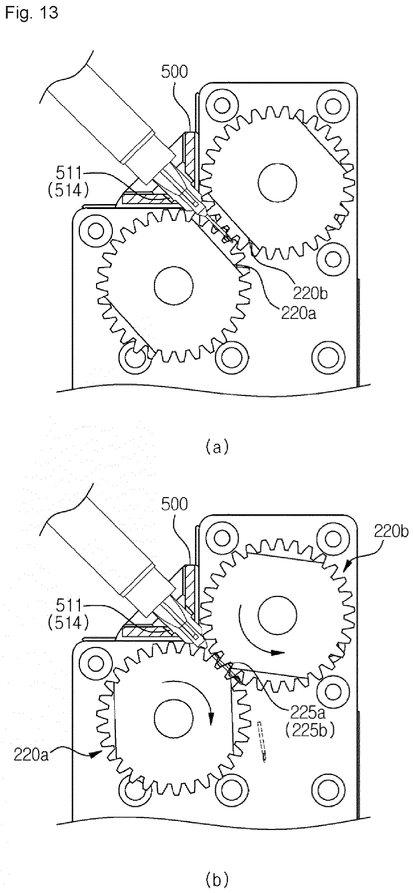

When the first and second non-engagement portions 220a and 220b for accommodation are formed, in the case of a needle having a normal length as shown in FIG. 12, the needle may be inserted into the empty space between the first and second non-engagement portions 220a and 220b for accommodation as much as possible, so that the separation time may be very short. In addition, in the case of a needle having a very short length as shown in FIG. 13, the needle may be separated by inserting the needle hub 2 into the empty space between the first and second non-engagement portions 220a and 220b for accommodation.

As shown in FIGS. 2 and 3, the first and second gears 200a and 200b may be rotatably supported on first and second gear support brackets 230a and 230b. The first and second gear support brackets 230a and 230b may be provided in pairs.

The first and second gear support brackets 230a and 230b may include first and second foot brackets 231a and 231b coupled to the bottom case 110, and first and second gear brackets 232a and 232b for rotatably supporting the first and second gears 200a and 200b.

A first rotation shaft (not shown) connected to an output shaft of a driving portion 300 may be installed to a center of the first gear 200a, and both ends of the first rotation shaft (not shown) may be rotatably supported between the first and second gear brackets 232a and 232b. The first rotation shaft (not shown) may be a driving shaft of the first gear 200a.

The second gear 200b may be rotatably supported between the first and second gear brackets 232a and 232b via a second rotation shaft (not shown). The second rotation shaft (not shown) may be composed of a bearing (not shown). The bearing (not shown) may be a driven shaft of the second gear 200b.

The first and second gear support brackets 230a and 230b may include a plurality of first and second cross bars 233a and 233b for holding a space between the first and second gear brackets 232a and 232b. The first and second cross bars 233a and 233b may can be fastened and fixed by fastening elements.

The first and second cross bars 233a and 233b may stably hold the first and second gears 200a and 200b without distortion, so that the second gear 200b may be prevented from deviation during separation of the used needle 3.

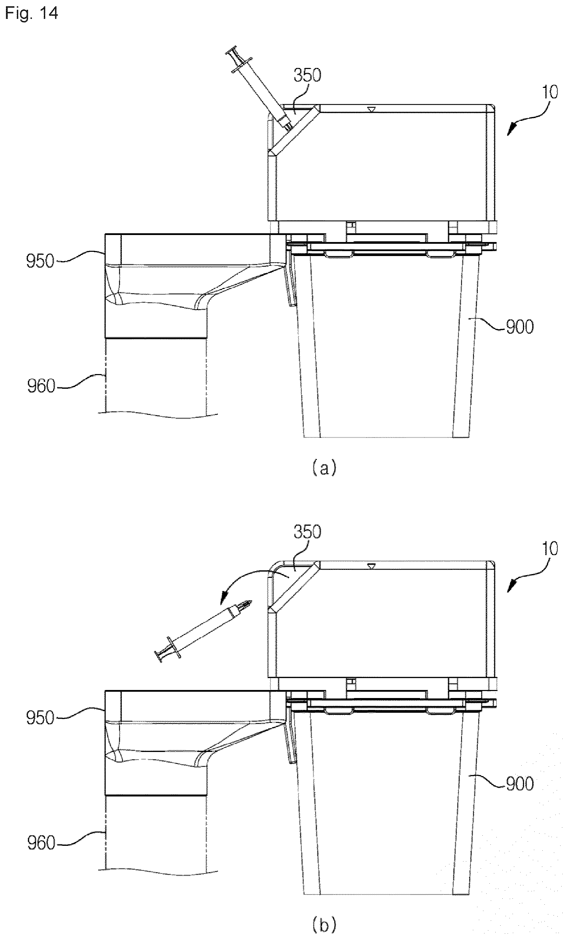

Referring to FIG. 3, the first and second rotation shafts (not shown) of the first and second gears 200a and 200b may be arranged at upper and lower parts parallel to each other, in which at least the first rotation shaft (not shown) may be arranged forward with respect to the second rotation shaft (not shown). The offset arrangement between the first and second rotation shafts (not shown), as shown in FIG. 14, may allow the needle 3 to be dropped by a weight of the used syringe immediately after being automatically separated when the used syringe is inserted into the insertion port 131 at a 45 degree angle and released.

Referring to FIGS. 2 and 3, the used needle separating device 10 according to the embodiment of the present invention may include a driving portion 300. The driving portion 300 may transmit rotational power to the first gear 200a.

The driving portion 300 may include a motor 310. The motor 310 may be supplied with power from a battery (not shown) installed in the bottom case 110 as in the present embodiment. The motor may also be supplied with external power.

The driving portion 300 may include a deceleration gear portion 320. The deceleration gear portion 320 may be supported on the first gear support bracket 230a. The deceleration gear portion 320 may support the motor 310. The deceleration gear portion 320 may decelerate rotation of the motor 310. An output shaft (not shown) of the deceleration gear portion 320 may be connected to the rotation shaft (not shown) of the first gear 200a. The deceleration gear portion 320 and the motor 310 may be placed and supported on the bottom case 110.

When the first start switch 330 or the second start switch 340 is operated, power may be applied to the driving portion 300 to drive the first and second gears 200a and 200b.

The first start switch 330 may be configured as a limit switch. A start button 350 may be installed on a lever of the first start switch 330. The start button 350 may be operated when the used syringe is inserted into the insertion port 131, pulled laterally and then pressed as shown in FIG. 14. In other words, the first start switch 330 may be applied when only the needle 3 is inserted into a small-diameter hole 513 as shown in FIG. 12(a). Because the needle 3 may be pulled laterally and bent, the first start switch 330 may be operated by using the syringe body.

The second start switch 340 may be directly pressed by the user to apply the power. The second start switch 340 may be configured as a push button switch. In other words, the second start switch 340 may be applied when the hub 2 is inserted into a large-diameter hole 513 or a cross hole 514 as shown in FIG. 13(a). Since the hub 2 is completely inserted into the large-diameter hole 513 or the cross hole 514 and accordingly difficult to be bent laterally like the needle 3, the start button 350 may not be operated by using the syringe body. Thus, the user is allowed to press the second start switch 340.

When the power to the driving portion 300 is cut off by a stop switch 360, the drives of the first and second gears 200a and 200b may be stopped. The stop switch 360 may be configured as a limit switch as shown in FIG. 3. The stop switch 360 may be restricted by a stop cam 370. The stop cam 370 may include a disc 371, touch protrusions 373 formed at 180-degree intervals around the disc 371, and a rotation shaft 375 formed at a rotation center of the disc 371. The touch protrusions 373 may be touched by a roller 363 attached to a lever 361 of the stop switch 360. The rotation shaft 375 may be inserted into a central rotation shaft of the first gear 200a through a hole 234 of the second gear bracket 232b.

The power off may be implemented as a mechanical mechanism as above, however, the electrical circuit configuration may also be used to stop the rotation of the first and second gears 200a and 200b after rotation at 180 degree angles.

In addition, a RED LED (not shown) around the second start switch 340 is turned on when the power is applied, and the RED LED (not shown) is turned off when the power is cut off, so that the user may check the operating status by using the eyes.

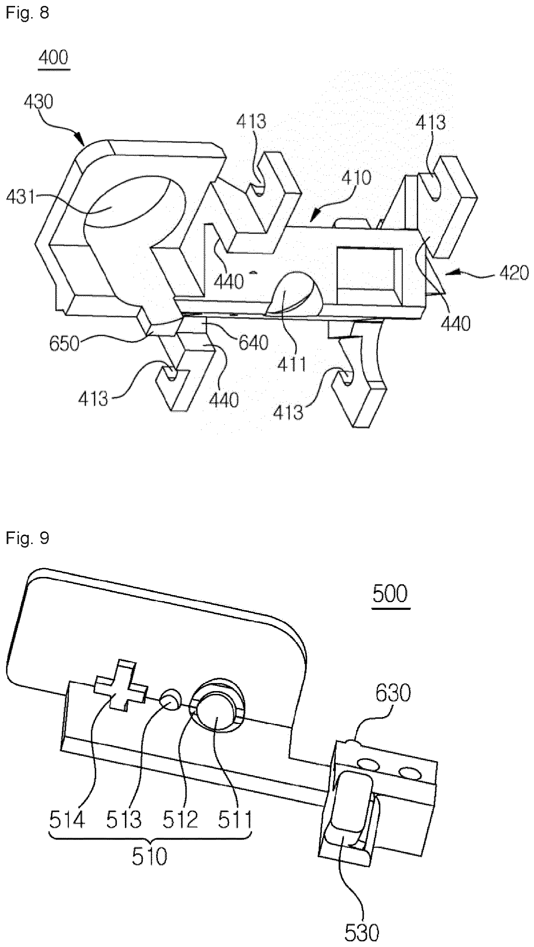

FIGS. 7 and 8 are front and rear perspective views showing a front and a rear of the holder portion 400.

Referring to FIGS. 3, 7 and 8, the used needle separating device 10 according to the embodiment of the present invention may include the holder portion 400. The holder portion 400 may hold the first start switch 330, the second start switch 340 and the guide jig portion 500. In particular, the holder portion 400 may support the guide jig portion 500 so as to be slid left and right.

The holder portion 400 may include a first holder 410. The first holder 410 may include a holder communication hole 411. The holder communication hole 411 may communicate with the insertion port 131. A holder support bracket 413 may be formed in the first holder 410. The holder support bracket 413 may be formed in four places on left, right, upper and lower sides of the first holder 410. As shown in FIG. 2, when the first and second cross bars 233a and 233b are fastened with screws, the holder support bracket 413 may be supported on the second gear support bracket 230b by pressure of screw heads.

The holder portion 400 may include a second holder 420. The second holder 420 may be formed on the left side of the first holder 410. The second holder 420 may include a cradle 421 to which the first start switch 330 is fastened, and a fitting groove 423 into which a part of the first start switch 330 is fitted. The second holder 420 may hold the first start switch 330 doubly.

The holder portion 400 may include a third holder 430. The third holder 430 may be formed on the right side of the first holder 410. The third holder 430 may include a holding through-hole 431 for holding the second start switch 340.

FIG. 9 is a front perspective view showing a guide jig portion 500.

Referring to FIGS. 3 and 9, the used needle separating device 10 according to the embodiment of the present invention may include a guide jig portion 500. The guide jig portion 500 may have a plate shape. The guide jig portion 500 may be slidably supported left and right on a rear surface of the holder portion 400. In addition, the guide jig portion 500 may guide a syringe use-specific needle hubs to be placed between the first and second non-engagement portions 220a and 220b for accommodation. In other words, the guide jig portion 500 may include a plurality of jig communication holes 510 into which the syringe use-specific needle hubs are inserted. The jig communication holes 510 may communicate with the holder communication hole 411.

The jig communication hole 510 may include a large-diameter hole 511 into which a cylindrical hub of a used needle is inserted, a small-diameter hole 513 into which only the needle of a used needle is inserted, and a cross hole 515 into which a cross hub of a used needle is inserted.

The large-diameter hole 511 may have a hole diameter, for example, of 4.6 mm, and may be used for an insulin syringe (having a very short length of needle) into which a needle hub of a used needle is inserted as shown in FIG. 13. A step 512 on which the syringe body is caught may be formed in the large-diameter hole 511. A diameter of the step 512 may be formed to be 6.6 mm.

The small-diameter hole 513 may have a hole diameter, for example, of 4.6 mm, and may be used as a general syringe in which only the needle of a used needle is inserted as shown in FIG. 12.

The cross hole 514 may be used for a syringe having a cross-shaped hub, such as a Luer lock syringe or a syringe having a short needle length of 10 mm or less. The cross hole 514 may only allow the insertion of a cross hub.

The guide jig portion 500 may include a grip portion 530. The grip portion 530 may be installed on a right front side of the guide jig portion 500 so as to be disposed on the long hole 134. The user may adjust the jig communication hole 510 to a position suitable for the use of the used needle by holding and sliding the grip portion 530.

Meanwhile, the first holder 410 of the holder portion 400 and the guide jig portion 500 may have a plate shape bent in a V shape. The V shape may allow the jig communication hole 510 to be positioned as close as possible to the engagement teeth portions of the first and second gears 200a and 200b, so that needle may be quickly separated. Accordingly, the first and second gear brackets 232a and 232b may be formed thereon with an L-shaped step to which the V-shaped guide jig portion 300 is seated (see FIG. 3).

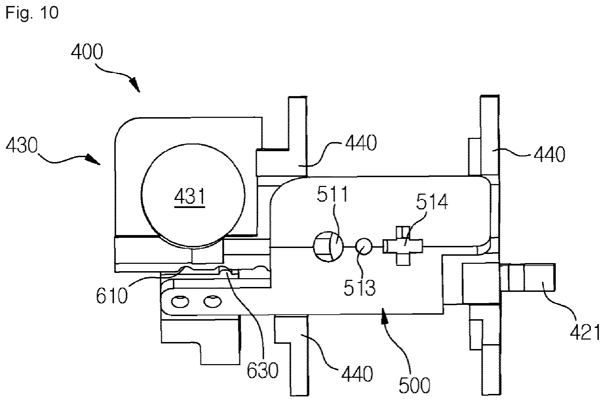

In addition, the V-shape may stably induce sliding of the guide jig portion 500 with respect to the holder portion 400. In other words, a plurality of support guide pieces 440 may be formed on upper and lower parts of the rear surface of the first holder 410. The support guide pieces 440 may guide and support upper and lower ends of the guide jig portion 500 so as to be slide left and right as shown in FIG. 10. In addition, the V shape allows the first holder 410 to support the guide jig portion 500 while bringing surface contact, so that stable sliding may be implemented.

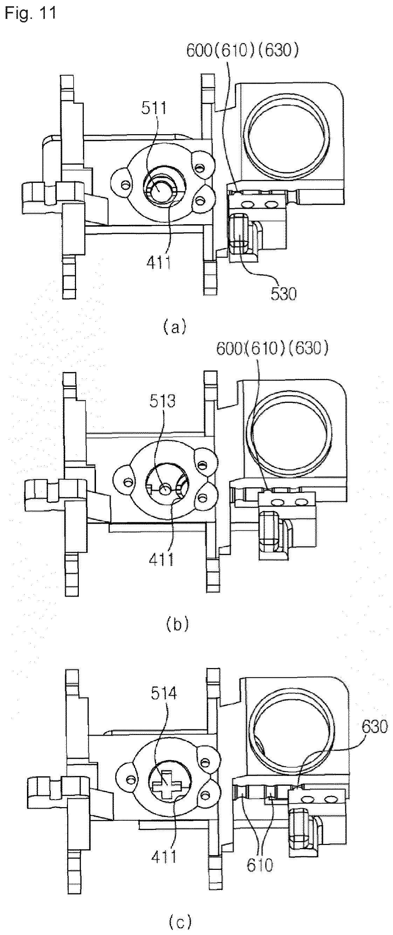

FIG. 10 is a rear view showing a state in which the holder portion 400 and the guide jig portion 500 are combined; and FIG. 11 shows front views of operating positions of the guide jig portion 500 with respect to the holder portion 400.

Referring to FIGS. 10 and 11, the used needle separating device 10 according to the embodiment of the present invention may include a positioning portion 600. The positioning portion 600 may determine a position of the guide jig portion 500 with respect to the holder portion 400. The positioning portion 600 may include a positioning groove 610 formed in the holder portion 400, and a positioning protrusion 630 formed in the guide jig portion 500. The positioning groove 610 and the positioning protrusion 630 can be formed in three stages.

In other words, the first, second and third stages may determine positions of the large-diameter hole 511, the small-diameter hole 513 and the cross hole 514 as shown in (a), (b) and (c) of FIG. 11.

The positioning portion 600 may include a first stage stopper 640 and a third stage stopper 650 as shown in FIG. 8. Accordingly, the guide jig portion 500 may slide between the first stage stopper 640 and the third stage stopper 650.

FIG. 15 is a sectional view showing an attachment/detachment operation between the needle collection container 800 and the bottom case 110.

Referring to FIGS. 1 and 15, the used needle separating device 10 according to the embodiment of the present invention may include the needle collection container 900. The needle collection container 900 may be a container in which the separated needles 3 are collected. The bottom case 110 of the case 100 may be coupled to an upper part of the needle collection container 900.

A first coupling portion 910 may be formed at a lower end of the bottom case 110, and a second coupling portion 930 to/from which the first coupling portion 910 is attached/detached may be formed in the needle collection container 900.

The first coupling portion 910 may include vertical coupling protrusions 911 protruding downward from left and right lower ends of the bottom case 110, horizontal coupling protrusions 913 each extending rearward from a lower end of the vertical coupling protrusion 911, and upward coupling protrusions 915 each protruding upward from a top surface of an end of the horizontal coupling protrusion 913.

The second connecting portion 930 may include horizontal protrusion frames 931 extending from left and right edges of the needle collection container 900, withdrawal holes 933 formed in the horizontal protrusion frame 931, and a downward coupling protrusion 935 protruding downward from a bottom surface of the horizontal protrusion frame 931 adjacent to the withdrawal hole 933.

Referring to solid arrows in FIG. 15, when the first coupling portion 910 is inserted into the withdrawal hole 933 downward from above and pulled rearward, the upper coupling protrusion 915 passes over the downward coupling protrusion 935 and is coupled. On the contrary, upon separation, the first coupling portion is pulled forward and lift upward in directions of dotted arrows.

A discharge hopper 950 may be detachably installed to the needle collection container 900. The discharge hopper 950 may be connected on a used syringe collection container (not shown) or through a bellows 960 as shown in FIG. 14. Then, as shown in FIG. 14(b), the used syringe separated from the needle 3 may be dropped into the discharge hopper 950 by its own weight and collected in the used syringe collection container (not shown).

Referring to the configurations of the above embodiment and FIGS. 12(a) and 13(b), the insertion port 131, the holder communication hole 411, and the jig communication hole 510 are arranged to communicate with each other in the space between the first and second non-engagement portions 220a and 220b for accommodation, and accordingly, the fixed portion of the needle as shown in FIG. 12(a) and the needle hub as shown in FIG. 13(a) are positioned as close as possible to the first and second engagement teeth 210a and 210b for separation, so that the needle separation time may be significantly shortened, and even very short needle can be separated while crushing the hub.

### Another Embodiment

A used needle separating device 1000 according to another embodiment of the present invention has a structure and a function almost similar to those of the used needle separating device 10 according to the embodiment of the present invention of FIG. 1, but differs from that the needle 3 of the blood collection-dedicated needle hub 1 (or catheter-dedicated needle hub) as shown in FIG. 24 is pulled out from the hub 2.

Accordingly, in the used needle separating device 1000 according to another embodiment of the present invention, parts identical in the used needle separating device 10 according to the embodiment of the present invention of FIG. 1 will be given the same reference numerals and detailed descriptions will be omitted.

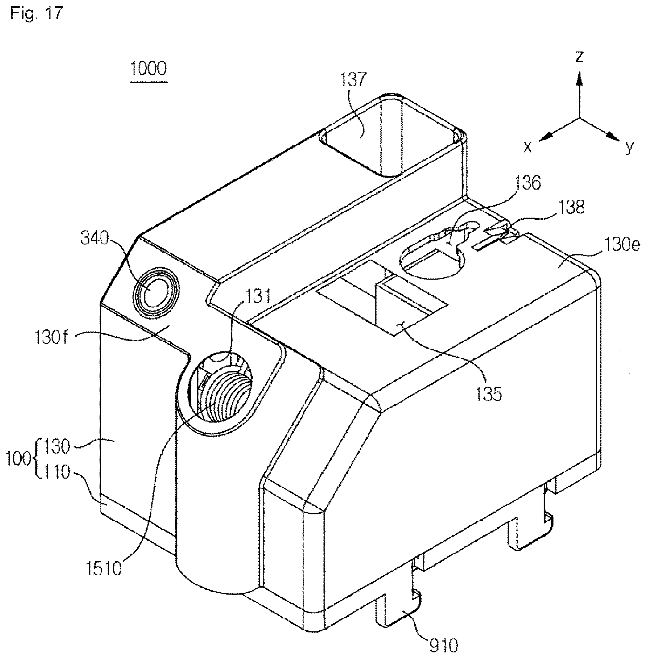

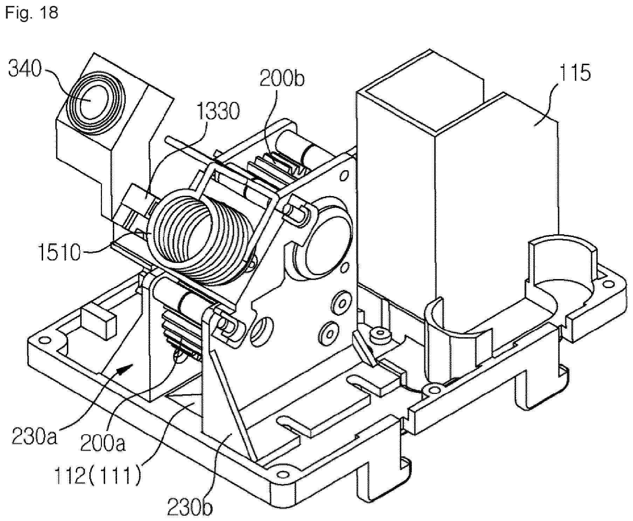

FIG. 17 is an exterior perspective showing the used needle separating device 1000 according to another preferred embodiment of the present invention; and FIG. 18 is a perspective view shown by removing the cover case of FIG. 17.

Referring to FIG. 17, the used needle separating device 1000 according to another embodiment of the present invention may also include a case 100 composed of a plate-shaped bottom case 110 and a cylinder-shaped cover case 130. The bottom case 110 may be coupled to the upper part of the needle collection container 900.

Referring to FIG. 18, a discharge hole 111 and an inclined surface 112 may be formed in the bottom case 110. The bottom case 110 may be coupled to the needle collection container 900 for collecting separated needles discharged through the discharge hole 111.

An insertion port 131 may be formed in the inclined plate 13f of the cover case 130. A needle hub 1 of the used needle may be inserted and pass through the insertion port 131. A second start switch 340 may be disposed in the inclined plate 130f.

The top plate 130e of the cover case 130 may be formed therein with a through-hole 135 disposed therein with a display window 810 of a counter 800, a manual needle separation penetration portion 136, a manual scalpel blade separation penetration portion 138, and a groove 137 for temporarily placing the used syringe having a needle which is not separated.

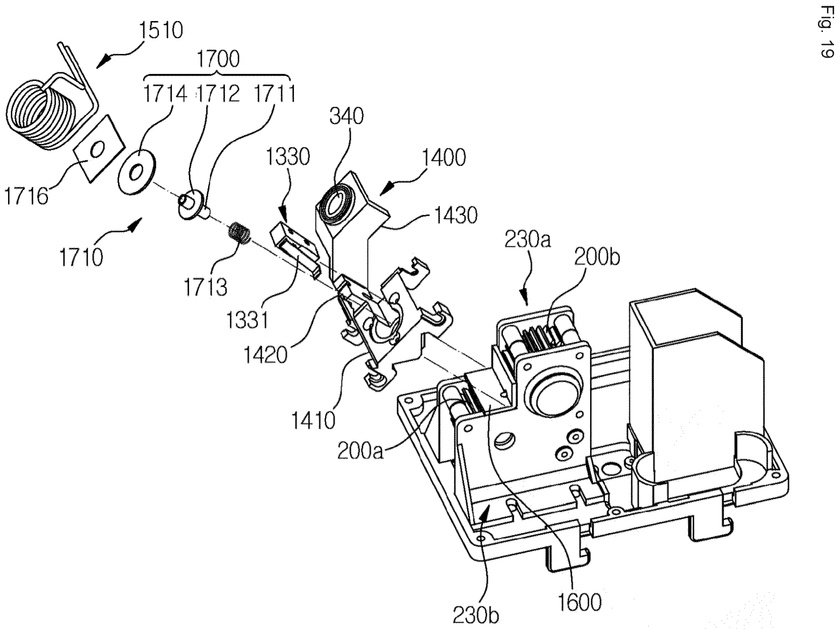

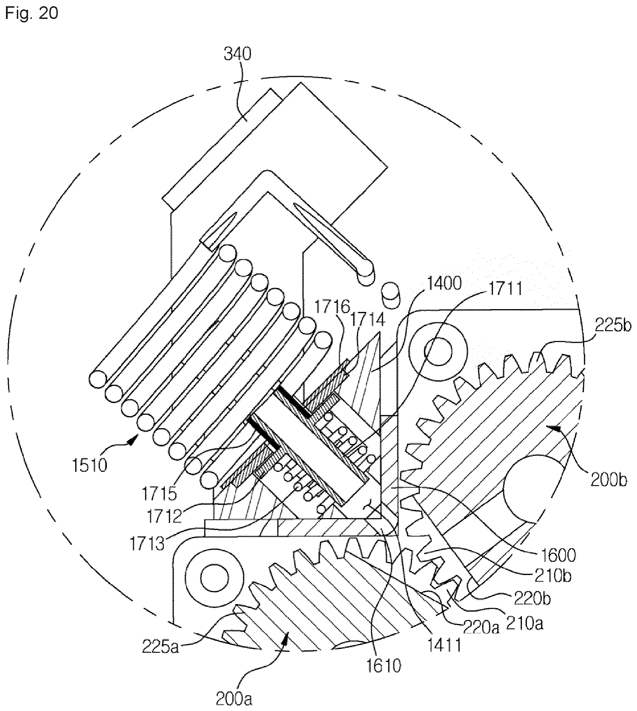

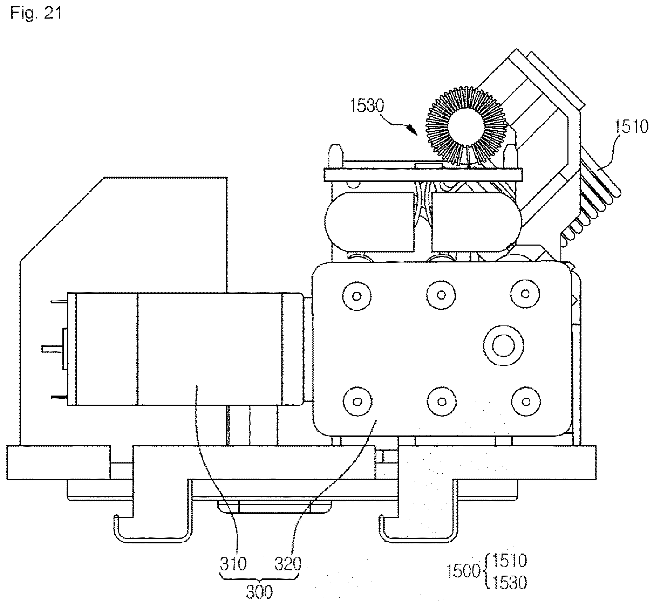

FIG. 19 is an exploded perspective view showing main parts of FIG. 18; FIG. 20 is a sectional view showing the main parts of FIG. 18; and FIG. 21 is a left side view of FIG. 18.

Referring to FIGS. 18 to 20, the used needle separating device 1000 according to another embodiment of the present invention may also include the first and second gears 200a and 200b for separating (pulling out) a used needle. The first and second gears 200a and 200b serving as the core of the present invention may also include, as shown FIG. 20, first and second engagement teeth 210a and 210b for drive rotation, first and second non-engagement portions 220a and 220b for accommodation and first and second engagement teeth 225a and 225b for separation. In addition, an empty space between the first and second non-engagement portions 220a and 220b for accommodation may be arranged to face the insertion port 131 to communicate with each other. The first and second gears 200a and 200b may be rotatably supported on first and second gear support brackets 230a and 230b coupled to the bottom case 110 as shown in FIGS. 18 and 19.

Referring to FIG. 21, the used needle separating device 1000 according to another embodiment of the present invention may also include a driving portion 300 for transmitting rotational force to the first gear 200a. The driving portion 300 may include a motor 310 and a deceleration gear portion 320.

When the first start switch 1330 or the second start switch 340 is operated, power may be supplied to the driving portion 300 to drive the first and second gears 200a and 200b.

The first start switch 1330 may be turned ON by touching a lever 1331 due to insertion force of the needle hub 1 inserted into the insertion port 131 as described later. In addition, the first start switch 1330 is configured as a switch that turns ON a high frequency induction coil heater 1500 described below and turns ON the driving portion 300 4 to 5 seconds later.

The second start switch 340 may be configured as a push button switch turned ON when being manually pressed by the user.

In addition, referring to FIG. 2, the driving portion 300 may be configured as an electric circuit that turns OFF the power of the driving portion 300 through a stop switch 360 and a stop cam 370 to stop the driving of the first and second gears 200a and 200b or stop the rotation of the first and second gears 200a and 200b after rotation by 180-degree angles.

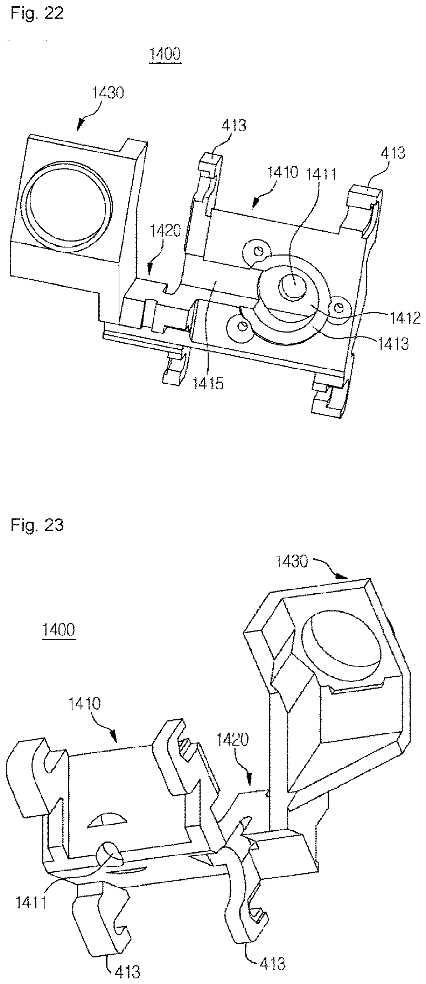

FIGS. 22 and 23 are front and rear perspective views showing a holder portion 1400 of FIG. 19.

Referring to FIGS. 22 and 23, the used needle separating device 1000 according to another embodiment of the present invention may include a holder portion 1400 supported by the gear support brackets 230a and 230b.

The holder portion 1400 may include a first holder 1410 in which a single holder communication hole 1411 is formed. The holder communication hole 1411 may communicate with the insertion port 131 so as to face each other. A holder support bracket 413 may be formed in the first holder 1410.

Referring to FIGS. 20 and 23, the first holder 1410 may have a plate shape bent into a V shape. The holder communication hole 1411 may be formed on a bent boundary line. Accordingly, the holder communication hole 1411 may be positioned as close as possible to the engagement teeth portions of the first and second gears 200a and 200b, so that needle may be quickly separated. Referring to FIG. 22, the first holder 1410 may include a latching plate 1412. The holder communication hole 1411 may be formed in the latching plate 1412. The first holder 1410 may include a seating groove 1413. The seating groove 1413 may be formed on a front periphery of the first holder 1410. The holder communication hole 1411, the latching plate 1412 and the seating groove 1413 may be arranged on the same center line. The first holder 1410 may include a horizontal groove 1415 communicating with a second holder 1420. In other words, the horizontal groove 1415 may be formed between the latching plate 1412 and the second holder 1420. A part of the first start switch 1330 may be fitted into the horizontal groove 1415, and a free end of the lever 1331 may be placed thereon.

The holder portion 1400 may include a second holder 1420 on which the first start switch 1330 is fastened and mounted.

The holder portion 1400 may include a third holder 1430 for holding the second start switch 340.

Referring to FIGS. 19 and 20, the used needle separating device 1000 according to another embodiment of the present invention may include an interposition portion 1600 formed of metal. The interposition portion 1600 may be disposed on a rear surface of the holder portion 1400 in a V shape. The interposition portion 1600 may include an interposition communication hole 1610 communicating with the holder communication hole 1411. Accordingly, the interposition portion 1600 may support the holder portion 1400 form of a plastic material to protect from the first and second gears 200a and 200b.

Referring to FIGS. 18 to 21, the used needle separating device 1000 according to another embodiment of the present invention may include a heating portion. The heating portion may transmit heat to an adhesive through the metal needle 7. In other words, the heating portion may heat the metal needle 7 by heating surroundings of the hub 6, and the heat may melt the adhesive so as to significantly reduce adhesion strength. The heating portion according to the present embodiment may include a high frequency induction coil heater 1500. When a needle hub 5 is placed inside a high frequency induction coil 1510 and a high frequency current is passed therethrough, the high frequency induction coil heater 1500 may generate an eddy current near a surface of a metal conductor to heat the metal conductor using heat due to the loss.

The high frequency induction coil heater 1500 may include the high frequency induction coil 1510. The high frequency induction coil 1510 may be disposed between the insertion port 131 and the holder communication hole 1411. The hub 6 may be disposed inside the high frequency induction coil 1510. Accordingly, the metal needle 7 may be heated using high frequency heat. The high frequency induction coil 1510 may be set to heat for 4 to 5 seconds.

The high frequency induction coil heater 1500 may include a high frequency induction heater 1530.

Referring to FIGS. 19 and 20, the used needle separating device 1000 according to another embodiment of the present invention may include a one-touch power actuator 1700.

The one-touch power actuator 1700 may sequentially apply power to the high frequency induction coil heater 1700 and the driving portion 300 by touching the first start switch 1330 while the needle hub 5 is inserted.

The one-touch power actuator 1700 may include the first start switch 1330 as described above.

The one-touch power actuator 1700 may include a touch operation portion 1710. The touch operation portion 1710 may be pressed by touching the lever 1331 of the first start switch 1330.

The touch operation portion 1710 may include a hollow shaft 1711. The needle 7 may be inserted into an inner side of the hollow shaft 1711. The hub 6 may be latched at the top of the hollow shaft 1771 so as to be pressed.

The touch operation portion 1710 may include a flange 1712. The flange 1712 may be formed on an outer peripheral surface about the middle of the hollow shaft 1711. A rear of the flange 1712 may be latched on the latching plate 1412.

The touch operation portion 1710 may include a spring 1713. The spring 1713 may be installed between the rear of the flange 1712 and the latching plate 1412.

The touch operation portion 1710 may include a latching hollow plate 1714. The latching hollow plate 1714 may have a washer shape. The latching hollow plate 1714 may be installed in a first holder portion 1410 while being seated in the seating groove 1413. The latching hollow plate 1714 may prevent the front of the flange 1712 from being latched and detached while the upper hollow shaft of the flange 1712 is exposed to the outside.

The touch operation portion 1710 may include a silicon bush 1715. The silicone bush 1715 may be fitted onto an outer peripheral surface of the upper hollow shaft of the flange 1712.

The touch operation portion 1710 may include a silicon hollow plate 1716. The silicone hollow plate 1716 may cover a front surface of the latching hollow plate 1714. The silicon hollow plate 1716 may be installed on the front surface of the first holder portion 1410, and may be fitted onto the outer peripheral surface of the upper hollow shaft of the flange 1712.

The silicone bush 1715 and the silicone hollow plate 1716 may minimize heat transmission to plastic parts during the high frequency heating.

It will be apparent to those skilled in the art that the present invention may be embodied in other specific forms without departing from the spirit and essential characteristics of the present invention. the above detailed description should not be construed as limiting in all aspects, but should be considered as illustrative. The scope of the present invention will be determined by a reasonable interpretation of the appended claims, and all changes within the equivalent scope of the present invention are included in the scope of the present invention.

### Description of Reference Numerals

10,1000: Used needle separating device
100: Case
131: (Needle hub) insertion port
200a,200b: First and second gears
210a,210b : First and second engagement teeth for drive rotation
220a,220b: First and second non-engagement portions for accommodation
225a,225b: First and second engagement teeth for separation
300: Driving portion
400,1400: Holder portion
411,1411: Holder communication hole
500: Guide jig portion
510: Jig communication hole
511: Large-diameter hole
513: Small-diameter hole
514: Cross hole
600: Positioning portion
610: Positioning groove
630: Positioning protrusion
700: Sterilizing lamp
800: Counter
900: Needle collection container
950: Discharge hopper
1500: High frequency induction coil heater
1510: High frequency induction coil
1530: High frequency induction heater
1700: One-touch power actuator
1710: Touch operation portion
1711: Hollow shaft
1712: Flange
1713: Spring
1714: Latching hollow plate
175: Silicone bush
1716: Silicone hollow plate

## Claims

1. A used needle separating device comprising:
a case formed therein with an insertion port for a used syringe needle;
first and second gears supported by a gear support bracket mounted on the case to separate the used syringe needle inserted into the insertion port; and
a drive portion for rotating the first and second gears, wherein
the first and second gears are formed along one circumference thereof with first and second engagement teeth for drive rotation, and formed along an opposite circumference thereof with first and second non-engagement portions for accommodating the used needle and first and second engagement teeth for engaging and separating the used needle.

2. The used needle separating device of claim 1, wherein the first non-engagement portion for accommodation is formed at 180 degree intervals on the first gear, and the second non-engagement portion for accommodation is formed at 180 degree intervals on the second gear.

3. The used needle separating device of claim 1, further comprising:
a holder portion supported by the gear support bracket and including a holder communication hole communicating with the insertion port; and
a guide jig portion slidably installed on a rear of the holder portion to guide a syringe use-specific needle hub to the first and second non-engagement portions for accommodation, wherein
the guide jig portion is formed therein with a plurality of jig communication holes to communicate between the holder communication hole and the first and second non-engagement portions for accommodation.

4. The used needle separating device of claim 3, wherein the holder portion and the guide jig portion are bent such that the holder communication hole and the jig communication hole are positioned close to an engaged part between the first and second gears.

5. The used needle separating device of claim 3, wherein the jig communication hole includes a large-diameter hole into which a cylindrical hub of the used needle is inserted, a small-diameter hole into which a needle of the used needle is inserted, and a cross hole into which a cross hub of the used needle is inserted.

6. The used needle separating device of claim 4, wherein the holder portion is formed on upper and lower parts of a rear thereof with support guide pieces for slidably supporting and guiding upper and lower ends of the guide jig portion, the holder portion is formed therein with a positioning groove for determining a position of the jig communication hole, and the guide jig portion is formed therein with a positioning protrusion latched in the positioning groove.

7. The used needle separating device of claim 1, wherein first and second rotation shafts of the first and second gears are arranged parallel to each other at upper and lower area, in which at least the first rotation shaft is disposed forward with respect to the second rotation shaft.

8. The used needle separating device of claim 7, wherein a start button for touching a first start switch of the driving portion protrudes from one side case for the insertion port, and a stop cam for touching a stop switch of the driving portion is installed on either of the first and second rotation shafts.

9. The used needle separating device of claim 8, wherein the holder portion includes a first holder formed therein with the holder communication hole, a second holder formed on one side of the first holder so that the first start switch is installed, and a third holder formed on an opposite side of the first holder and installed therein with a second start switch,
the start button is installed on a lever of the first start switch,
the start button retractably protrudes from a through-hole formed in the one side case for the insertion port,
the positioning groove is formed on a lower side of the third holder,
the positioning protrusion is formed on a rear of a grip portion installed in the guide jig portion, and
the grip portion protrudes from a long hole formed on a front surface of the case.

10. The used needle separating device of claim 5, wherein the case is assembled on an upper part of the needle collection container for collecting the used needle, the case is formed therein with a discharge hole for dropping the separated needle into the needle collection container, and the needle collection container is installed therein with a discharge hopper for guiding a used syringe dropped by an own weight to the used syringe collection container.

11. The used needle separating device of claim 1, wherein the case is assembled on an upper part of the needle collection container for collecting the used needle, the case is installed therein with a sterilizing lamp for irradiating light to an inside of the needle collection container, and the case is installed therein with a counter for counting the used needle.

12. The used needle separating device of claim 1, further comprising:
a holder portion supported by the gear support bracket and including a holder communication hole communicating with the insertion port; and
a heating portion installed between the insertion port and the holder communication hole to heat the used needle.

13. The used needle separating device of claim 12, wherein the heating portion includes a high frequency induction coil heater, and the high frequency induction coil heater includes a high frequency induction coil installed between the above insertion port and the holder communication hole, and a high frequency induction heater for heating the high frequency induction coil.

14. The used needle separating device of claim 12, wherein the holder portion is installed therein with a one-touch power actuator, and the one-touch power actuator includes a first start switch installed on one side of the holder communication hole, and a touch operation portion for touching the lever of the first start switch.

15. The used needle separating device of claim 14, wherein the touch operation portion includes a hollow shaft arranged between the insertion port and the holder communication hole, a flange formed on an outer peripheral surface between two ends of the hollow shaft to touch the lever, a spring installed between a rear of the flange and the holder communication hole, and a latching hollow plate installed in the holder portion to allow a front of the flange to be latched while exposing the upper hollow shaft of the flange.

16. The used needle separating device of claim 15, wherein a silicone bush is fitted on an outer peripheral surface of the upper hollow shaft, and the holder portion is installed therein with a silicone hollow plate for covering the front of the latching hollow plate.
